# EUROPEAN PATENT SPECIFICATION

(11) **EP 3 049 381 B1**
(45) Date of publication and mention of the grant of the patent: **08.08.2018**
(21) Application number: 14849603.7
(22) Date of filing: 28.08.2014
(51) Int. Cl.: C07C 7/12, C07C 15/08, C07C 6/12, C07C 7/00, C07C 7/04, C07C 7/11, B01D 3/14

(54) **APPARATUSES AND METHODS FOR ISOLATING C8 AROMATICS**
VORRICHTUNG UND VERFAHREN ZUR ISOLIERUNG VON C8-AROMATEN
APPAREILS ET PROCÉDÉS POUR ISOLER DES AROMATIQUES C8

(30) Priority: 27.09.2013 US 201314040318
(43) Date of publication of application: 03.08.2016
(73) Proprietor: UOP LLC, Des Plaines, Illinois 60017-5017 (US)
(72) Inventor: GATTUPALLI, Rajeswar, Des Plaines, Illinois 60017-5017 (US); CORRADI, Jason T., Des Plaines, Illinois 60017-5017 (US); WHITCHURCH, Patrick, Des Plaines, Illinois 60017-5017 (US); WERBA, Gregory, Des Plaines, Illinois 60017-5017 (US)
(74) Representative: Boult Wade Tennant
(86) International application number: PCT/US2014/053035
(87) International publication number: WO 2015/047644

(56) References cited:
- WO-A2-2006/138063
- US-A- 3 522 153
- US-A- 3 584 068
- US-A1- 2010 168 347
- US-A1- 2010 305 380
- US-A1- 2012 247 943
- US-A1- 2012 271 084
- US-A1- 2013 153 503
- US-A1- 2013 158 330

## Description

### STATEMENT OF PRIORITY

This application claims priority to U.S. Application No. 14/040,318 which was filed September 27, 2013.

### TECHNICAL FIELD

The present disclosure generally relates to apparatuses and methods for processing hydrocarbons during the production of desired isomers of xylene, and more particularly relates to apparatuses and methods for isolating aromatic hydrocarbons having eight carbon atoms (C8).

### BACKGROUND

Xylenes are aromatic hydrocarbons that include a benzene ring and two methyl substituents. Based on the structural position of the methyl substituents, three isomers of xylene can be formed: paraxylene, metaxylene, and orthoxylene. Paraxylene is a feedstock for terephthalic acid, which is used in the manufacture of synthetic fibers and resins. Metaxylene is used in the manufacture of certain plasticizers, azo dyes, and wood preservatives. Orthoxylene is a feedstock for phthalic anhydride, which is used in the manufacture of certain plasticizers, dyes, and pharmaceutical products.

For production of a desired xylene isomer, a mixed stream of the three xylene isomers is typically produced before the desired xylene isomer is separated. In other words, the desired xylene is not selectively produced but is selectively separated. A desired xylene isomer can be separated from mixed xylene streams by using an adsorbent selective to the desired isomer. After the desired isomer is adsorbed from the mixed xylene stream, the remaining isomers are discharged in a mixed raffinate stream. Typically, a deadsorbent desorbs the desired xylene isomer from the adsorbent, and the deadsorbent and selected xylene isomer are collected and separated by fractionation.

In the production of paraxylene, heavy deadsorbents are conventionally used to desorb the paraxylene from the adsorbent. Heavy deadsorbents are defined as having higher molecular weights and higher boiling points than xylene. Accordingly, light deadsorbents are defined as having lower molecular weights and lower boiling points than xylene. Heretofore, xylene isomer recovery systems using heavy deadsorbents have typically required less energy than systems with light deadsorbents, because the heavy deadsorbent does not require repeated evaporation and lifting during fractionation. However, heavy deadsorbent systems typically require stringent feed purity to control accumulation of undesired compounds in the recycled deadsorbent, such as impurities that reduce deadsorbent effectiveness and product purity. Further, additional equipment may be required to maintain heavy deadsorbent purity during the deadsorbent recycling process. Also, systems using heavy deadsorbent have fractionation columns with relatively higher reboiler temperatures. Higher reboiler temperatures lead to higher operating pressures that require higher pressure ratings for the equipment involved, thereby increasing the equipment capital cost.

Use of a light deadsorbent, such as the relatively inexpensive light deadsorbent toluene, relaxes feed specifications relative to systems using heavy deadsorbent. Cost savings for the relaxed feed specifications can offset the increased energy costs associated with recovering light deadsorbent as a fractionation column overhead. Xylene recovery apparatuses using light deadsorbent also provide savings in the total equipment count as deadsorbent purification and storage units are not necessary. Further, xylene recovery apparatuses using light deadsorbent have lower fractionation column operating pressures, allowing for less expensive thinner column shells with lower pressure ratings.

To efficiently produce a selected xylene isomer from a hydrocarbon stream using light deadsorbent, it is desirable to separate substantially all of the aromatic hydrocarbons having eight carbon atoms (C8), including xylene and ethylbenzene, from the hydrocarbon stream and from recycled portions of the stream during processing. Heretofore, xylene recovery apparatuses utilizing light deadsorbent have not efficiently isolated aromatic C8 for xylene recovery.
US 3, 584,068 describes a process for C8 aromatic feed fractionation.
US 3,522,153 describes a method of separating xylene isomers by distillation with crystallization and isomerization of a side stream.
US 2010/0168347 describes processes using dividing wall distillation columns.
US 2010/0305380 describes a process for improved meta-xylene yield from C8 aromatics.
US2012/0271084 describes the recycle of transalkylation effluent fractions enriched in trimethylbenzene.

Accordingly, it is desirable to provide methods and apparatuses for isolating aromatic C8 from hydrocarbon streams. Also, it is desirable to provide methods and apparatuses that separate aromatic C8 from hydrocarbon streams by first removing aromatic C8 in a sidedraw fraction and a bottom fraction, and by then removing the aromatic C8 as an overhead from the bottom fraction. In addition, it is desirable to develop methods and apparatuses for efficiently producing selected xylene isomers from hydrocarbon streams. Furthermore, other desirable features and characteristics of the present embodiment will become apparent from the subsequent detailed description and the appended claims, taken in conjunction with the accompanying drawings and this background.

### BRIEF SUMMARY

Apparatuses and methods are provided for isolating C8 aromatics from hydrocarbon streams. In an exemplary embodiment, a method for separating C8 aromatics from a hydrocarbon stream includes introducing the hydrocarbon stream to a fractionation column at a feed point. Further, the method includes fractionating the hydrocarbon stream in the fractionation column. Also, the method includes withdrawing a sidedraw fraction from the fractionation column at a draw point located above the feed point, wherein the sidedraw fraction includes C8 aromatics.

In another embodiment, a method for isolating C8 aromatics is provided and includes fractionating a hydrocarbon stream including C8 aromatics into an overhead fraction including C7⁻ hydrocarbons, a sidedraw fraction including a portion of the C8 aromatics, and a bottom fraction including remaining C8 aromatics and C8⁺ hydrocarbons. The method further includes fractioning the bottom fraction and forming a heavy overhead fraction including the remaining C8 aromatics. The method combines the sidedraw fraction and the heavy overhead fraction to isolate the C8 aromatics.

An apparatus for separating C8 aromatics from a hydrocarbon stream is also provided. The apparatus includes a first fractionation column configured to receive the hydrocarbon stream and to fractionate the hydrocarbon stream into a sidedraw fraction including a portion of the C8 aromatics and a bottom fraction including the remaining C8 aromatics and C8⁺ hydrocarbons. Further, the apparatus includes a second fractionation column configured to receive the bottom fraction and to fractionate the bottom fraction into a heavy overhead fraction including the remaining C8 aromatics.

### BRIEF DESCRIPTION OF THE DRAWINGS

The present embodiment will hereinafter be described in conjunction with the following drawing figures, wherein like numerals denote like elements, and wherein:
FIG. 1 is a schematic diagram of an exemplary embodiment of a method and an apparatus for isolating aromatic C8 from a hydrocarbon stream; and
FIG. 2 is a schematic diagram of the method and the apparatus of FIG. 1 in use in a scheme for producing a selected xylene isomer product.

### DETAILED DESCRIPTION

The following detailed description is merely exemplary in nature and is not intended to limit the application and uses of the embodiment described. Furthermore, there is no intention to be bound by any theory presented in the preceding background or the following detailed description.

The various embodiments described herein relate to apparatuses and methods for separating aromatic C8 from hydrocarbon streams. Isolation of aromatic C8 may provide for enhanced production of a selected xylene isomer from a mixed xylene feedstock. As described below, a hydrocarbon stream is fractionated to produce a sidedraw fraction of a mixed xylene stream with aromatic hydrocarbons containing 8 carbon atoms (aromatic C8). Further, the fractionation process forms a bottom fraction containing a remaining portion of the aromatic C8. The bottom fraction is fractionated to form a heavy overhead fraction containing the remaining aromatic C8. In this manner, the aromatic C8 is efficiently isolated from the hydrocarbon stream. Further, the combined stream formed from the sidedraw fraction and the heavy overhead fraction has a sufficient aromatic C8 composition to allow for efficient separation of a selected xylene isomer in further downstream processing. As used herein, the phrase "overhead fraction" is not limited to the uppermost fraction from a fractionation process or apparatus, but may include the uppermost fraction and/or any fraction formed above the sidedraw and bottom fraction. Further, as used herein, the phrase "bottom fraction" is not limited to the lowermost fraction from a fractionation process or apparatus, but may include the lowermost fraction and/or any fraction formed below the sidedraw and overhead fraction.

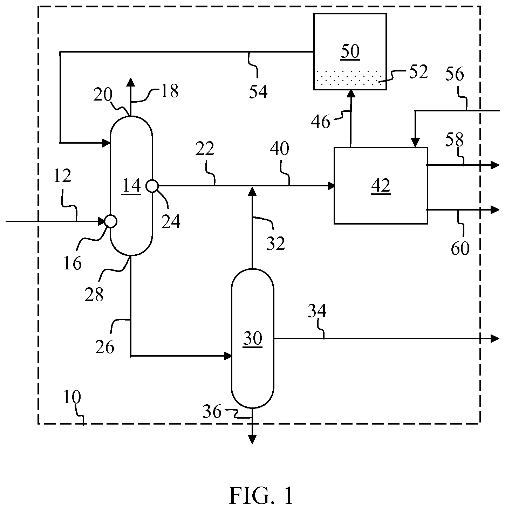

Reference is now made to an exemplary embodiment of an apparatus 10 for isolating C8 aromatics in FIG. 1. A hydrocarbon stream 12 is fed to a fractionation unit 14, such as a stripper column, at a feed point 16. An exemplary hydrocarbon stream 12 has a relatively high concentration of aromatic compounds, such as 40 to 100 mass percent. Suitable hydrocarbon streams12 are available from many sources. For example, a fluid catalytic cracking (FCC) unit and fractionator run in high severity mode can produce a fraction with hydrocarbons having 7 to 10 carbon atoms (C7-10), where 60 mass percent of the hydrocarbons are aromatic. Certain coal liquefaction processes produce hydrocarbon streams rich in aromatic compounds, and these hydrocarbon streams are suitable for use as hydrocarbon stream 12. Other possible sources include various petroleum refining processes, thermal or catalytic cracking of hydrocarbons, or petrochemical conversion processes, including hydrocarbon streams processed in a reformer using a catalyst designed to produce aromatic compounds. Additional processing steps (not illustrated in FIG. 1) can be used to remove non-aromatic compounds from the hydrocarbon stream 12 in some embodiments, such as liquid liquid extraction, extractive crystallization, clay treating, or additional fractionation.

The fractionation unit 14 is operated at conditions suitable for forming an overhead fraction 18 primarily containing hydrocarbons having seven and fewer carbon atoms (C7⁻) that exits the fractionation unit 14 at or around its top 20. An exemplary overhead fraction 18 contains more than 80%, for example more than 90%, such as more than 95%, hydrocarbons having seven and fewer carbon atoms. The fractionation unit 14 further forms a sidedraw fraction 22 primarily containing aromatic hydrocarbons having eight carbon atoms (C8) that exits the fractionation unit 14 at a draw point 24. An exemplary sidedraw fraction 22 is rich in aromatic C8 and contains more than 80%, for example more than 90%, such as more than 95%, or more than 98%, aromatic hydrocarbons having eight carbon atoms. The sidedraw fraction 22 may be considered to form a mixed xylene stream. The fractionation unit 14 also forms a bottom fraction 26 primarily containing hydrocarbons having eight and more carbon atoms (C8⁺) that exits from the fractionation unit 14 at or around its bottom 28. An exemplary bottom fraction 26 contains more than 80%, for example more than 90%, such as more than 95%, hydrocarbons having eight and more carbon atoms.

The different fractions (such as C7⁻, C8, and C8⁺) are separated based on the relative boiling points of the compounds present. To provide desired separation, the fractionation unit 14 can be operated from a pressure of 5 kiloPascals absolute (kPa) to 1,800 kPa (0,7 pounds per square inch absolute (PSIA) to 260 PSIA), and a temperature from 35°C to 360°C (65°F to 680°F).

As shown, the draw point 24 is located above the feed point 16, i.e., between the feed point 16 and the top 20 of the fractionation unit 14. Likewise, the feed point 16 is located below the draw point 24, i.e., between the draw point 24 and the bottom 28 of the fractionation unit 14. In an exemplary embodiment, the fractionation unit 14 is formed with trays and the draw point 24 is located at a higher tray than the feed point 16. By providing the draw point 24 above the feed point 16, recovery of relatively heavier species, such as hydrocarbons having nine or more carbon atoms (C9⁺) in the sidedraw fraction 22 is inhibited.

In FIG. 1, the bottom fraction 26, containing C8⁺ species including some aromatic C8, is introduced to a heavy aromatics fractionation unit 30. The heavy aromatics fractionation unit 30 is operated at conditions suitable for forming a heavy overhead fraction 32 that contains substantially all of the aromatic C8 that is introduced into the heavy aromatics fractionation unit 30 in the bottom fraction 26. The heavy overhead fraction 32 may be considered to form a mixed xylene stream. The heavy aromatics fractionation unit 30 also forms a heavy sidedraw fraction 34 that contains aromatic hydrocarbons having nine or ten carbon atoms (C9-C10) and a heavy bottom fraction 36 that contains hydrocarbons having eleven and more carbon atoms (C11⁺). The heavy sidedraw fraction 34 and the heavy bottom fraction 36 may exit the apparatus 10 for further processing.

The different fractions (such as C8, C9-C10, and C11⁺) are separated in the heavy aromatics fractionation unit 30 based on the relative boiling points of the compounds present. The heavy aromatics fractionation unit 30 can be operated from a pressure of 5 kPa to 1800 kPa (0.7 PSIA to 260 PSIA), and a temperature from 100°C to 360°C (212°F to 680°F).

The mixed xylene streams, i.e., the sidedraw fraction 22 from the fractionation unit 14 and the heavy overhead fraction 32 from the heavy aromatics fractionation unit 30, are combined to form a combined mixed xylene stream 40. In an exemplary embodiment, the combined mixed xylene stream 40 is further processed to isolate a selected xylene isomer. Therefore, the combined mixed xylene stream 40 is introduced into a separation unit 42 that separates a selected xylene isomer from non-selected xylene isomers. An exemplary separation unit 42 includes a selective adsorbent that preferentially sorbs the selected xylene isomer relative to the other xylene isomers. A deadsorbent is then used to desorb the selected xylene isomer from the adsorbent, and the deadsorbent and selected xylene isomer are collected and separated by distillation. In an exemplary embodiment, the selective adsorbent is crystalline alumino- silicate, such as type X or type Y crystalline aluminosilicate zeolites. The exemplary selective adsorbent contains exchangeable cationic sites with one or more metal cations, where the metal cations can be one or more of lithium, potassium, beryllium, magnesium, calcium, strontium, barium, nickel, copper, silver, manganese, and cadmium. Sorption conditions vary, but typically range from 35°C to 200°C (100°F to 400°F) and from 100 kPa to 3,500 kPa (14 PSIA to 500 PSIA).

Separation of the selected xylene isomer from the non-selected xylene isomers results in the formation of a raffinate xylene isomer stream 46 containing the non-selected xylene isomers. In the exemplary apparatus 10, the raffinate xylene isomer stream 46 is fed to an isomerization unit 50 where the non-selected xylene isomers are isomerized to produce more of the selected xylene isomer. Specifically, the removal of the selected xylene isomer in the separation unit 42 shifts the composition of the raffinate xylene isomer stream 46 away from the equilibrium between isomer species. Because the raffinate xylene isomer stream 46 primarily includes the non-selected two of the three xylene isomers and is relatively deficient in the selected xylene isomer, the selected xylene isomer is produced in the isomerization unit 50 to bring the xylene isomers closer to an equilibrium ratio. At 250°C, the equilibrium ratio is 20 to 25 percent orthoxylene, 20 to 30 percent paraxylene, and 50 to 60 percent metaxylene, though the equilibrium ratio varies with temperature and other conditions.

In an exemplary embodiment, the isomerization unit 50 includes an isomerization catalyst 52, and operates at suitable isomerization conditions. Suitable isomerization conditions include a temperature from 100°C to 500°C (200°F to 900°F), or from 200°C to 400°C (400°F to 800°F), and a pressure from 500 kPa to 5,000 kPa ( 70 PSIA to 700 PSIA). The isomerization unit 50 includes a sufficient volume of isomerization catalyst to provide a liquid hourly space velocity, with respect to the raffinate xylene isomer stream 46, from 0.5 to 50 hr⁻¹, or from 0.5 to 20 hr⁻¹. Hydrogen may be present at up to 15 moles of hydrogen per mole of xylene, but in some embodiments hydrogen is essentially absent from the isomerization unit 50. The isomerization unit 50 may include one, two, or more reactors, where suitable means are employed to ensure a suitable isomerization temperature at the entrance to each reactor. The xylenes are contacted with the isomerization catalyst in any suitable manner, including upward flow, downward flow, or radial flow.

An exemplary isomerization catalyst includes a zeolitic aluminosilicate with a Si:Al₂ ratio greater than 10/1, or greater than 20/1 in some embodiments, and a pore diameter of 5 to 8 angstroms. Some examples of suitable zeolites include, but are not limited to, MFI, MEL, EUO, FER, MFS, MTT, MTW, TON, MOR, and FAU, and gallium may be present as a component of the crystal structure. In some embodiments, the Si:Ga₂ mole ratio is less than 500/1, or less than 100/1 in other embodiments. The proportion of zeolite in the catalyst is generally from 1 weight percent (wt%) to 99 wt%, or from 25 wt% to 75 wt%. In some embodiments, the isomerization catalyst includes 0.01 wt% to 2 wt% of one or more of ruthenium (Ru), rhodium (Rh), palladium (Pd), osmium (Os), Iridium (Ir), and platinum (Pt), but in other embodiments the isomerization catalyst is substantially absent of any metallic compound, where substantial absence is less than 0.01 wt%. The balance of the isomerization catalyst is an inorganic oxide binder, such as alumina, and a wide variety of catalyst shapes can be used, including spherical or cylindrical.

An isomerized stream 54 with an equilibrium distribution of xylene isomers exits the isomerization unit 50 and is recycled to the fractionation unit 14. The xylenes in the isomerized stream 54 continue on to the separation unit 42 via the sidedraw fraction 22 or the heavy overhead fraction 32. In the exemplary apparatus 10, the isomerized stream 54 is passed through the fractionation unit 14 so that C8 compounds that were changed to a compound with a different number of carbon atoms in the isomerization unit 50 can be removed via fractions 18, 34 or 36. The isomerized stream 54 includes more of the selected xylene isomers than the raffinate xylene isomer stream 46, so more of the selected xylene isomer is available for recovery in the separation unit 42. As a result, the amount of the recovered selected xylene isomer can exceed its theoretical equilibrium value at the processing temperatures.

Separation of the selected xylene isomer from the non-selected xylene isomers in the separation unit 42 further results in the formation of an extract stream (not illustrated) containing the selected xylene isomer and the deadsorbent. Within the separation unit 42, the deadsorbent 56 is used to desorb the selected xylene isomer from the adsorbent. The deadsorbent 56 and the selected xylene isomer will form an extract stream, which is fed to an extract column (not shown). The deadsorbent 56 is then separated from the selected xylene isomer by fractionation in an extract column (not shown) in the separation unit 42. The selected xylene isomer exits the extract column as a bottoms stream, which, if required can be sent to a finishing column to further purify the selected xylene stream to meet product quality specifications. The selected xylene stream leaves the finishing column as an overhead fraction and is discharged from the separation unit 42 as product stream 58. Product stream 58 can be removed from the apparatus 10 as the selected xylene product, e.g., a paraxylene product, an orthoxylene product, or a metaxylene product. The bottoms stream from the finishing column may include some selected xylene isomer and is discharged from the separation unit 42 as stream 60.

Several different embodiments of the separation unit 42 are possible, such as a single bed operated in batch fashion, where the raffinate xylene isomer stream 46 is collected before the selected xylene isomer is desorbed, and the extract stream is collected after desorbing. In another embodiment, multiple adsorbent beds are used, and the introduction point of the combined mixed xylene stream 40 and the deadsorbent 56 are gradually moved through the different adsorbent beds. The discharge points of the extract stream and the raffinate xylene isomer stream 46 are also gradually moved through the different adsorbent beds, so each individual adsorbent bed is used in a semi-batch mode and the combination simulates a continuous operation. As a light deadsorbent, deadsorbent 56 has a lower molecular weight than xylene as well as a deadsorbent boiling point lower than the selected xylene isomer boiling point or the non-selected xylene isomer(s) boiling point.

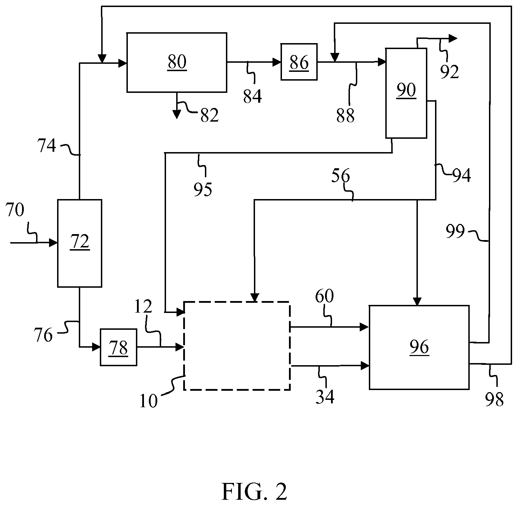

Referring to FIG. 2, the apparatus 10 is shown in integration with other hydrocarbon processing scheme. As shown, the hydrocarbon stream 12 is formed from a feed stream 70. An exemplary feed stream 70 is a naphtha feedstock. Naphtha feedstocks include aromatics, paraffins, and naphthenes, and may include small amounts of olefins. Feedstocks which may be utilized include straight-run naphthas, natural gasoline, synthetic naphthas, thermal gasoline, catalytically cracked gasoline, and in particular reformed naphthas. The feedstock may be encompassed by a full-range naphtha as defined by boiling points, or from 0° to 230°C, or naphthas having a greater percentage, such as greater than 50% or greater than 70%, of aromatic hydrocarbons.

As shown in FIG. 2, the feed stream 70, particularly in embodiments where the feed stream 70 is a reformed naphtha stream, is fed to a reformate splitter distillation column 72. The reformate splitter distillation column 72 functions to separate or "split" by distilling the feed stream 70 into a lower boiling stream as an overhead stream 74 and a higher boiling stream as a bottom stream 76. The reformate splitter distillation column may be configured such that, for example, the overhead stream 74 may include primarily (such as greater than 80%, greater than 90%, or greater than 95%) hydrocarbons having seven or fewer carbon atoms (C7). The bottom stream 76 may thus include primarily, such as greater than 80%, greater than 90%, or greater than 95%, hydrocarbons having eight or more carbon atoms (C8⁺).

The bottom stream 76 may thereafter be passed to a clay treater 78 for the removal of any alkylates and olefins that may be present in the bottom stream 76. The clay treater 78 may be configured in any known manner suitable for this purpose. The hydrocarbon stream 12 leaving the clay treater 78 may thus include primarily, such as greater than 80%, greater than 90%, or greater than 95%, C8⁺ hydrocarbons with alkylate and olefin compounds substantially, such as greater than 90%, removed therefrom.

The overhead stream 74 is passed from the reformate splitter distillation column 72 to an extractive distillation process unit 80 for removing non-aromatic compounds from the overhead stream 74. In one particular embodiment, extractive distillation process unit 80 may employ a sulfolane solvent to separate aromatic compounds from non-aromatic compounds. Other extraction methods, such as liquid-liquid solvent extraction are also well-known and practiced for separation of non-aromatic compounds from aromatic compounds, and their use in place of, or in addition to, extractive distillation process unit 80 is contemplated herein. Extractive distillation process unit 80 produces a stream 82 that includes primarily, such as greater than 80%, greater than 90%, or greater than 95%, non-aromatic C7⁻ hydrocarbons and a stream 84 that includes primarily, such as greater than 80%, greater than 90%, or greater than 95%, benzene and toluene. Stream 84 may further be passed to a clay treater 86 for increasing the purity of the aromatic compounds in such stream, for example by removing any alkylates or olefins that may be present therein in a manner as described above with regard to clay treater 78, thus producing a treated benzene and toluene stream 88.

The treated benzene and toluene stream 88 is thereafter passed to a split shell distillation column 90 for the separation of the benzene from the toluene in the treated benzene and toluene stream 88. The benzene, having a lower boiling point than toluene, is removed from distillation column 90 as an overhead product 92, and the toluene, having a higher boiling point than benzene, is removed from distillation column 90 as a sidedraw product 94. Also, a net bottoms liquid stream 95 including heavier aromatic hydrocarbons such as various xylene isomers, is removed from the distillation column 90 and thereafter fed to the apparatus 10, and more specifically to the fractionation unit 14 of FIG. 1.

The toluene in the sidedraw product 94 may be fed to the apparatus 10, and more specifically to the adsorbent chamber in the separation unit 42 as deadsorbent 56. Alternatively or additionally, the sidedraw product 94 is fed to a transalkylation transalkylation process unit 96. As shown, the transalkylation process unit 96 also receives the heavy sidedraw fraction 34 that contains aromatic hydrocarbons having nine or ten carbon atoms (C9-C10) and exits the heavy aromatics fractionation unit 30 (see FIG. 1) in the apparatus 10. Also, the transalkylation process unit 96 receives the stream 60 that may include some selected xylene isomer and that exits the finishing column in the separation unit 42 (see FIG. 1) of apparatus 10.

The transalkylation process unit 96 converts the toluene into benzene and xylenes in a toluene disproportionation process. Further, the transalkylation process unit 96 converts a mixture of toluene and aromatic hydrocarbons having nine carbon atoms (C9) into xylenes in a transalkylation process. Hydrogen is fed to the transalkylation process unit 96 so that the disproportionation and transalkylation processes are conducted in a hydrogen atmosphere to minimize coke formation. As shown, a stream 98 including benzene and toluene exits the transalkylation process unit 62 and may thereafter be passed to the extractive distillation process unit 80 for removing any non-aromatic compounds therein formed during the disproportionation and transalkylation processes. Also, a stream 99 of toluene and xylenes exits the transalkylation process unit 62 and is fed to the split shell distillation column 90 for the separation of toluene from the xylenes.

As described herein, an apparatus and method for isolating aromatic hydrocarbons having eight carbon atoms (C8) are provided. The apparatus uses two fractionation units to separate aromatic C8 from other hydrocarbons. Specifically, a first column forms an aromatic C8-rich sidedraw fraction and a bottom fraction including a remaining portion of aromatic C8. The bottom fraction is fed to a heavy aromatic fractionation column where the remaining aromatic C8 is separated in an overhead fraction and can be combined with the C8-rich sidedraw fraction. Further processing may be performed to provide a selected xylene isomer product from the combined stream of aromatic C8.

### SPECIFIC EMBODIMENTS

While the following is described in conjunction with specific embodiments, it will be understood that this description is intended to illustrate and not limit the scope of the preceding description and the appended claims.

A first embodiment of the invention is a method for separating C8 aromatics from a hydrocarbon stream, the method comprising the steps of introducing the hydrocarbon stream to a fractionation column at a feed point; fractionating the hydrocarbon stream in the fractionation column; and withdrawing a sidedraw fraction from the fractionation column at a draw point located above the feed point, wherein the sidedraw fraction includes C8 aromatics. An embodiment of the invention is one, any or all of prior embodiments in this paragraph up through the first embodiment in this paragraph further comprising withdrawing an overhead fraction from the fractionation column, wherein the overhead fraction includes C7⁻ hydrocarbons; and withdrawing a bottom fraction from the fractionation column, wherein the bottom fraction includes C9+ aromatics. An embodiment of the invention is one, any or all of prior embodiments in this paragraph up through the first embodiment in this paragraph further comprising withdrawing a bottom fraction from the fractionation column, wherein the bottom fraction includes C9+ aromatics and C8 aromatics; separating the bottom fraction into a heavy overhead fraction including C8 aromatics; and combining the heavy overhead fraction with the sidedraw fraction. An embodiment of the invention is one, any or all of prior embodiments in this paragraph up through the first embodiment in this paragraph further comprising separating the bottom fraction into a heavy bottom fraction including C11+ aromatics. An embodiment of the invention is one, any or all of prior embodiments in this paragraph up through the first embodiment in this paragraph further comprising separating the bottom fraction into a heavy sidedraw fraction including C9 aromatics and C10 aromatics. An embodiment of the invention is one, any or all of prior embodiments in this paragraph up through the first embodiment in this paragraph further comprising disproportionating and transalkylating toluene with the C9 aromatics and C10 aromatics in the heavy sidedraw fraction to produce benzene and xylene. An embodiment of the invention is one, any or all of prior embodiments in this paragraph up through the first embodiment in this paragraph further comprising withdrawing a bottom fraction from the fractionation column, wherein the bottom fraction includes C9- aromatics and C8 aromatics; separating the bottom fraction into a heavy sidedraw fraction including C9 aromatics and C10 aromatics; and disproportionating and transalkylating toluene with the C9 aromatics and C10 aromatics in the heavy sidedraw fraction to produce benzene and xylene. An embodiment of the invention is one, any or all of prior embodiments in this paragraph up through the first embodiment in this paragraph further comprising separating a reformed naphtha feedstream into an overhead portion including C7 and a bottom portion including C8⁺ aromatics, wherein the bottom portion forms the hydrocarbon stream. An embodiment of the invention is one, any or all of prior embodiments in this paragraph up through the first embodiment in this paragraph further comprising contacting the sidedraw fraction with an adsorbent configured to adsorb a selected xylene isomer from the sidedraw fraction; and contacting the adsorbent with a deadsorbent to desorb the selected xylene isomer from the adsorbent. An embodiment of the invention is one, any or all of prior embodiments in this paragraph up through the first embodiment in this paragraph further comprising contacting the sidedraw fraction with a adsorbent configured to adsorb paraxylene from the sidedraw fraction; and contacting the adsorbent with a deadsorbent to desorb the paraxylene from the adsorbent. An embodiment of the invention is one, any or all of prior embodiments in this paragraph up through the first embodiment in this paragraph further comprising withdrawing a bottom fraction from the fractionation column, wherein the bottom fraction includes C9+ aromatics and C8 aromatics; separating the bottom fraction into a heavy overhead fraction including C8 aromatics; combining the heavy overhead fraction with the sidedraw fraction to form a combined stream of C8 aromatics; contacting the combined stream of C8 aromatics with an adsorbent configured to adsorb a selected xylene isomer from the combined stream of C8 aromatics; and contacting the adsorbent with a deadsorbent to desorb the selected xylene isomer from the adsorbent.

A second embodiment of the invention is a method for isolating C8 aromatics, the method comprising the steps of fractionating a hydrocarbon stream including C8 aromatics into an overhead fraction including C7⁻ hydrocarbons, a sidedraw fraction including a portion of the C8 aromatics, and a bottom fraction including remaining C8 aromatics and C8⁺ hydrocarbons; fractioning the bottom fraction and forming a heavy overhead fraction including the remaining C8 aromatics; and combining the sidedraw and the heavy overhead fraction to isolate the C8 aromatics. An embodiment of the invention is one, any or all of prior embodiments in this paragraph up through the second embodiment in this paragraph wherein fractioning the bottom fraction comprises forming a heavy sidedraw fraction including C9 aromatics and C10 aromatics. An embodiment of the invention is one, any or all of prior embodiments in this paragraph up through the second embodiment in this paragraph further comprising disproportionating and transalkylating toluene with the C9 aromatics and C10 aromatics in the heavy sidedraw fraction fraction to produce benzene and xylene. An embodiment of the invention is one, any or all of prior embodiments in this paragraph up through the second embodiment in this paragraph wherein fractioning the bottom fraction comprises forming a heavy bottom fraction including C11+ aromatics. An embodiment of the invention is one, any or all of prior embodiments in this paragraph up through the second embodiment in this paragraph wherein fractionating a hydrocarbon stream including C8 aromatics into an overhead fraction, a sidedraw fraction, and a bottom fraction comprises feeding the hydrocarbon stream into a fractionation unit at a feed point and withdrawing the sidedraw fraction from the fractionation unit at a draw point above than the feed point. An embodiment of the invention is one, any or all of prior embodiments in this paragraph up through the second embodiment in this paragraph further comprising separating a reformed naphtha feedstream into an overhead portion including C7 and a bottom portion including C8⁺ aromatics, wherein the bottom portion forms the hydrocarbon stream. An embodiment of the invention is one, any or all of prior embodiments in this paragraph up through the second embodiment in this paragraph wherein combining the sidedraw fraction and the heavy overhead fraction comprises forming a combined stream, and wherein the method further comprises the step of contacting the combined stream with an adsorbent configured to adsorb a selected xylene isomer from the combined stream; and contacting the adsorbent with a deadsorbent to desorb the selected xylene isomer from the adsorbent. An embodiment of the invention is one, any or all of prior embodiments in this paragraph up through the second embodiment in this paragraph wherein combining the sidedraw fraction and the heavy overhead fraction comprises forming a combined stream, and wherein the method further comprises the step of contacting the combined stream with an adsorbent configured to adsorb paraxylene from the combined stream; and contacting the adsorbent with a deadsorbent to desorb the paraxylene from the adsorbent.

A third embodiment of the invention is an apparatus for separating C8 aromatics from a hydrocarbon stream, the apparatus comprising a first fractionation column configured to receive the hydrocarbon stream and to fractionate the hydrocarbon stream into a sidedraw fraction including a portion of the C8 aromatics and a bottom fraction including remaining C8 aromatics and C8⁺ hydrocarbons; and a second fractionation column configured to receive the bottom fraction and to fractionate the bottom fraction into a heavy overhead fraction including the remaining C8 aromatics.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the application in any way. Rather, the foregoing detailed description will provide those skilled in the art with a convenient road map for implementing one or more embodiments, it being understood that various changes may be made in the function and arrangement of elements described in an exemplary embodiment without departing from the scope, as set forth in the appended claims.

## Claims

1. A method for separating C8 aromatics from a hydrocarbon stream (12), the method comprising the steps of:
introducing the hydrocarbon stream to a fractionation column (14) at a feed point (16);
fractionating the hydrocarbon stream in the fractionation column; and
withdrawing a sidedraw fraction (22) from the fractionation column at a draw point (24) located above the feed point, wherein the sidedraw fraction includes C8 aromatics;
withdrawing a bottom fraction (26) from the fractionation column, wherein the bottom fraction includes C9+ aromatics and C8 aromatics;
separating the bottom fraction into a heavy bottom fraction (36) including C11+ aromatics, a heavy sidedraw fraction (34) including C9 aromatics and C10 aromatics, and a heavy overhead fraction (32) including C8 aromatics; and
combining the heavy overhead fraction with the sidedraw fraction.

2. The method of claim 1 further comprising:
withdrawing an overhead fraction (18) from the fractionation column, wherein the overhead fraction includes C7⁻ hydrocarbons.

3. The method of claim 1 further comprising disproportionating and transalkylating toluene (56) with the C9 aromatics and C10 aromatics in the heavy sidedraw fraction to produce benzene and xylene (98).

4. The method of claim 1 further comprising:
withdrawing a bottom fraction (26) from the fractionation column, wherein the bottom fraction includes C9+ aromatics and C8 aromatics;
separating the bottom fraction into a heavy sidedraw fraction (34) including C9 aromatics and C10 aromatics; and
disproportionating and transalkylating toluene (56) with the C9 aromatics and C10 aromatics in the heavy sidedraw fraction to produce benzene and xylene (98).

5. The method of claim 1 further comprising separating a reformed naphtha feedstream (70) into an overhead portion (74) including C7 and a bottom portion (76) including C8⁺ aromatics, wherein the bottom portion forms the hydrocarbon stream.

6. The method of claim 1 further comprising:
contacting the sidedraw fraction with an adsorbent configured to adsorb a selected xylene isomer from the sidedraw fraction; and
contacting the adsorbent with a deadsorbent (56) to desorb the selected xylene isomer from the adsorbent.

7. The method of claim 1 further comprising:
contacting the sidedraw fraction with an adsorbent configured to adsorb paraxylene from the sidedraw fraction; and
contacting the adsorbent with a deadsorbent (56) to desorb the paraxylene from the adsorbent.

8. The method of claim 1 further comprising:
withdrawing a bottom fraction (26) from the fractionation column, wherein the bottom fraction includes C9+ aromatics and C8 aromatics;
separating the bottom fraction into a heavy overhead fraction (32) including C8 aromatics;
combining the heavy overhead fraction with the sidedraw fraction to form a combined stream (40) of C8 aromatics;
contacting the combined stream of C8 aromatics with an adsorbent configured to adsorb a selected xylene isomer from the combined stream of C8 aromatics; and
contacting the adsorbent with a deadsorbent (56) to desorb the selected xylene isomer from the adsorbent.

9. An apparatus (10) for separating C8 aromatics from a hydrocarbon stream (12), the apparatus comprising:
a first fractionation column (14) configured to receive the hydrocarbon stream and to fractionate the hydrocarbon stream into a sidedraw fraction (22) including a portion of the C8 aromatics and a bottom fraction (26) including remaining C8 aromatics and C8⁺ hydrocarbons; and
a second fractionation column (30) configured to receive the bottom fraction and to fractionate the bottom fraction into a heavy overhead fraction (32) including the remaining C8 aromatics;
wherein the overhead fraction (32) is in fluid communication with the sidedraw fraction (22) of the first fractionation column (14).

## Patentansprüche

1. Verfahren zum Abtrennen von C8-Aromaten aus einem Kohlenwasserstoffstrom (12), wobei das Verfahren die Schritte umfasst:
Einführen des Kohlenwasserstoffstroms in eine Fraktionierkolonne (14) an einer Zuführstelle (16);
Fraktionieren des Kohlenwasserstoffstroms in der Fraktionierkolonne; und
Entnehmen einer Seitenstromfraktion (22) aus der Fraktionierkolonne an einer Entnahmestelle (24), die über der Zuführstelle angeordnet ist, wobei die Seitenstromfraktion C8-Aromaten umfasst;
Entnehmen einer Sumpffraktion (26) aus der Fraktionierkolonne, wobei die Sumpffraktion (C9+)-Aromaten und C8-Aromaten umfasst;
Auftrennen der Sumpffraktion in eine schwere Sumpffraktion (36), die (C11+)-Aromaten enthält, eine schwere Seitenstromfraktion (34), die C9-Aromaten und C10-Aromaten enthält, und eine schwere Kopffraktion (32), die C8-Aromaten enthält; und
Kombinieren der schweren Kopffraktion mit der Seitenstromfraktion.

2. Verfahren gemäß Anspruch 1, ferner umfassend:
Entnehmen einer Kopffraktion (18) aus der Fraktionierkolonne, wobei die Kopffraktion C7-Kohlenwasserstoffe enthält.

3. Verfahren gemäß Anspruch 1, ferner umfassend Disproportionieren und Transalkylieren von Toluol (56) mit den C9-Aromaten und C10-Aromaten in der schweren Seitenstromfraktion, um Benzol und Xylol (98) zu erzeugen.

4. Verfahren gemäß Anspruch 1, ferner umfassend:
Entnehmen einer Sumpffraktion (26) aus der Fraktionierkolonne, wobei die Sumpffraktion (C9+)-Aromaten und C8-Aromaten enthält;
Auftrennen der Sumpffraktion in eine schwere Seitenstromfraktion (34), die C9-Aromaten und C10-Aromaten enthält; und
Disproportionieren und Transalkylieren von Toluol (56) mit den C9-Aromaten und C10-Aromaten in der schweren Seitenstromfraktion, um Benzol und Xylol (98) zu erzeugen.

5. Verfahren gemäß Anspruch 1, ferner umfassend Auftrennen eines reformierten Naphtha-Zustroms (70) in einen Kopfteil (74), der C7 enthält, und einen Sumpfteil (76), der (C8+)-Aromaten enthält, wobei der Sumpfteil den Kohlenwasserstoffstrom bildet.

6. Verfahren gemäß Anspruch 1, ferner umfassend:
Inkontaktbringen der Seitenstromfraktion mit einem Adsorptionsmittel, das dafür gestaltet ist, ein ausgewähltes Xylol-Isomer aus der Seitenstromfraktion zu adsorbieren; und
Inkontaktbringen des Adsorptionsmittels mit einem Desorptionsmittel (56), um das ausgewählte Xylol-Isomer von dem Adsorptionsmittel zu desorbieren.

7. Verfahren gemäß Anspruch 1, ferner umfassend:
Inkontaktbringen der Seitenstromfraktion mit einem Adsorptionsmittel, das dafür gestaltet ist, para-Xylol aus der Seitenstromfraktion zu adsorbieren; und
Inkontaktbringen des Adsorptionsmittels mit einem Desorptionsmittel (56), um das para-Xylol von dem Adsorptionsmittel zu desorbieren.

8. Verfahren gemäß Anspruch 1, ferner umfassend:
Entnehmen einer Sumpffraktion (26) aus der Fraktionierkolonne, wobei die Sumpffraktion (C9+)-Aromaten und C8-Aromaten umfasst;
Auftrennen der Sumpffraktion in eine schwere Kopffraktion (32), die C8-Aromaten enthält;
Kombinieren der schweren Kopffraktion mit der Seitenstromfraktion, um einen kombinierten Strom (40) von C8-Aromaten zu bilden;
Inkontaktbringen des kombinierten Stroms von C8-Aromaten mit einem Adsorptionsmittel, das dafür gestaltet ist, ein ausgewähltes Xylol-Isomer aus dem kombinierten Strom von C8-Aromaten zu adsorbieren; und
Inkontaktbringen des Adsorptionsmittels mit einem Desorptionsmittel (56), um das ausgewählte Xylol-Isomer von dem Adsorptionsmittel zu desorbieren.

9. Vorrichtung (10) zum Abtrennen von C8-Aromaten aus einem Kohlenwasserstoffstrom (12), wobei die Vorrichtung umfasst:
eine erste Fraktionierkolonne (14), die dafür gestaltet ist, den Kohlenwasserstoffstrom aufzunehmen und den Kohlenwasserstoffstrom in eine Seitenstromfraktion (22), die einen Teil der C8-Aromaten enthält, und eine Sumpffraktion (26), die die restlichen C8-Aromaten und (C8+)-Kohlenwasserstoffe enthält, zu fraktionieren; und
eine zweite Fraktionierkolonne (30), die dafür gestaltet ist, die Sumpffraktion aufzunehmen und die Sumpffraktion in eine schwere Kopffraktion (32), die die restlichen C8-Aromaten enthält, zu fraktionieren;
wobei die Kopffraktion (32) in Fluidverbindung mit der Seitenstromfraktion (22) der ersten Fraktionierkolonne (14) steht.

## Revendications

1. Procédé de séparation d'aromatiques en C8 à partir d'un flux d'hydrocarbures (12), le procédé comprenant les étapes de :
introduction du flux d'hydrocarbures dans une colonne de fractionnement (14) à un point d'alimentation (16) ;
fractionnement du flux d'hydrocarbures dans la colonne de fractionnement ; et
prélèvement d'une fraction de prélèvement latéral (22) de la colonne de fractionnement à un point de prélèvement (24) situé au-dessus du point d'alimentation, dans lequel la fraction de prélèvement latéral comprend des aromatiques en C8 ;
prélèvement d'une fraction de queue (26) de la colonne de fractionnement, dans lequel la fraction de queue comprend des aromatiques en C9+ et des aromatiques en C8 ;
séparation de la fraction de queue en une fraction de queue lourde (36) comprenant des aromatiques en C11+, une fraction de prélèvement lourde (34) comprenant des aromatiques en C9 et des aromatiques en C10, et une fraction de tête lourde (32) comprenant des aromatiques en C8 ; et
combinaison de la fraction de tête lourde avec la fraction de prélèvement latéral.

2. Procédé selon la revendication 1 comprenant en outre :
le prélèvement d'une fraction de tête (18) de la colonne de fractionnement, la fraction de tête comprenant des hydrocarbures en C7⁻.

3. Procédé selon la revendication 1, comprenant en outre la dismutation et la transalkylation de toluène (56) avec les aromatiques en C9 et les aromatiques en C10 dans la fraction de prélèvement latéral lourde pour produire du benzène et du xylène (98).

4. Procédé selon la revendication 1 comprenant en outre :
le prélèvement d'une fraction de queue (26) de la colonne de fractionnement, dans laquelle la fraction de queue comprend des aromatiques en C9 + et des aromatiques en C8 ;
la séparation de la fraction de queue en une fraction de prélèvement latéral lourde (34) comprenant des aromatiques en C9 et des aromatiques en C10 ; et
la dismutation et la transalkylation de toluène (56) avec les composés aromatiques en C9 et les composés aromatiques en C10 dans la fraction de prélèvement latéral lourde pour produire du benzène et du xylène (98) .

5. Procédé selon la revendication 1, comprenant en outre la séparation d'un flux d'alimentation de naphta reformé (70) en une partie de tête (74) comprenant C7 et une partie de queue (76) comprenant des aromatiques en C8⁺, dans lequel la partie de queue forme le flux d'hydrocarbures.

6. Procédé selon la revendication 1 comprenant en outre :
la mise en contact de la fraction de prélèvement latéral avec un adsorbant configuré pour adsorber un isomère de xylène sélectionné à partir de la fraction de prélèvement latéral ; et
la mise en contact de l'adsorbant avec un désorbant (56) pour désorber l'isomère de xylène sélectionné de l'adsorbant.

7. Procédé selon la revendication 1 comprenant en outre :
la mise en contact de la fraction de prélèvement latéral avec un adsorbant configuré pour adsorber le paraxylène à partir de la fraction de prélèvement latéral ; et
la mise en contact de l'adsorbant avec un désorbant (56) pour désorber le paraxylène de l'adsorbant.

8. Procédé selon la revendication 1 comprenant en outre :
le prélèvement d'une fraction de queue (26) à partir de la colonne de fractionnement, dans lequel la fraction de queue comprend des aromatiques en C9+ et des aromatiques en C8 ;
la séparation de la fraction de queue en une fraction de tête lourde (32) comprenant des aromatiques en C8 ;
la combinaison de la fraction de tête lourde avec la fraction de prélèvement latéral pour former un flux combiné (40) d'aromatiques en C8 ;
la mise en contact du flux combiné d'aromatiques en C8 avec un adsorbant configuré pour adsorber un isomère de xylène sélectionné à partir du flux combiné d'aromatiques en C8 ; et
la mise en contact de l'adsorbant avec un désorbant (56) pour désorber l'isomère de xylène sélectionné de l'adsorbant.

9. Appareil (10) pour séparer les aromatiques en C8 d'un flux d'hydrocarbures (12), l'appareil comprenant :
une première colonne de fractionnement (14) configurée pour recevoir le flux d'hydrocarbures et pour fractionner le flux d'hydrocarbures en une fraction de prélèvement latéral (22) comprenant une partie des composés aromatiques en C8 et une fraction de queue (26) comprenant des aromatiques en C8 et des hydrocarbures en C8⁺ restants ; et
une deuxième colonne de fractionnement (30) configurée pour recevoir la fraction de queue et pour fractionner la fraction de queue en une fraction de tête lourde (32) comprenant les aromatiques en C8 restants ;
dans lequel la fraction de tête (32) est en communication fluidique avec la fraction de prélèvement latéral (22) de la première colonne de fractionnement (14) .
